(19) Europäisches Patentamt / European Patent Office / Office européen des brevets

(11) **EP 3 998 249 B1**

(12) **EUROPEAN PATENT SPECIFICATION**

(45) Date of publication and mention of the grant of the patent:
**01.01.2025  Bulletin 2025/01**

(21) Application number: **21818350.7**

(22) Date of filing: **03.06.2021**

(51) International Patent Classification (IPC):
**C07C 67/08** *(2006.01)*    **C07C 69/82** *(2006.01)*

(52) Cooperative Patent Classification (CPC):
(C-Sets available)
**C07C 67/08**                                        (Cont.)

(86) International application number:
**PCT/KR2021/006961**

(87) International publication number:
**WO 2021/246809 (09.12.2021 Gazette 2021/49)**

(54) **REACTION CONTROL METHOD IN CONTINUOUS MANUFACTURING PROCESS OF DIESTER-BASED COMPOSITION**

REAKTIONSSTEUERUNGSVERFAHREN FÜR EIN KONTINUIERLICHES HERSTELLUNGSVERFAHREN EINER ZUSAMMENSETZUNG AUF DIESTERBASIS

PROCÉDÉ DE COMMANDE DE RÉACTION DANS UN PROCÉDÉ DE FABRICATION EN CONTINU D'UNE COMPOSITION À BASE DE DIESTER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2020  KR 20200068019**

(43) Date of publication of application:
**18.05.2022  Bulletin 2022/20**

(73) Proprietor: **LG Chem, Ltd.**
**Seoul 07336 (KR)**

(72) Inventors:
• **CHOO, Yeon Uk**
  **Daejeon 34122 (KR)**
• **JEONG, Jae Hun**
  **Daejeon 34122 (KR)**
• **LEE, Sung Kyu**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
CN-A- 107 032 985    CN-U- 203 971 948
JP-A- H01 071 840    JP-A- H02 306 936
JP-A- H06 247 899    KR-B1- 101 663 586
KR-B1- 101 663 586   US-A1- 2014 288 325

(52) Cooperative Patent Classification (CPC): (Cont.)

C-Sets
**C07C 67/08, C07C 69/82**

**Description**

**TECHNICAL FIELD**

**Cross-reference to Related Applications**

This application claims the benefit of Korean Patent Application No. 10-2020-0068019, filed on June 5, 2020, in the Korean Intellectual Property Office.

**Technical Field**

The present invention relates to a control method by which a conversion rate in a reactor may reach a target conversion rate in a continuous production process of a diester-based composition.

**BACKGROUND ART**

Phthalate-based plasticizers had occupied 92% of the world's plasticizer market by the 20th century (Mustafizur Rahman and Christopher S.Brazel "The plasticizer market: an assessment of traditional plasticizers and research trends to meet new challenges" Progress in Polymer Science 2004, 29, 1223- 1248), and are additives used to improve the processability of polyvinyl chloride (hereinafter, referred to as PVC) by imparting flexibility, durability, cold resistance, and the like and lowering viscosity during melting. Phthalate-based plasticizers are introduced into PVC in various contents and used not only for hard products such as rigid pipes, but also for soft products such as food packaging materials, blood bags, and flooring materials since the phthalate-based plasticizers are soft and stretchable. Thus, the phthalate-based plasticizers are more closely related to real life than any other materials and are widely used for materials which come into direct contact with a human body.

However, despite the compatibility with PVC and excellent softness imparting properties of phthalate-based plasticizers, there has been controversy over the harmful nature of the phthalate-based plasticizers in that when a PVC product containing a phthalate-based plasticizer is used in real life, the phthalate-based plasticizer may be leaked little by little out of the product and act as a suspected endocrine disruptor (environmental hormone) and a carcinogen to the level of a heavy metal (NR Janjua et al. "Systemic Uptake of Diethyl Phthalate, Dibutyl Phthalate, and Butyl Paraben Following Whole-body Topical Application and Reproductive and Thyroid Hormone Levels in Humans" Environmental Science and Technology 2007, 41, 5564-5570). Particularly, since a report was published in the 1960s in the United States that diethylhexyl phthalate (di-(2-ethylhexyl) phthalate, DEHP), the most used phthalate plasticizer, leaked out of PVC products, global environmental regulations have started to be implemented in addition to various studies on the harmful nature of the phthalate-based plasticizer on human bodies, boosted by increasing interest in environmental hormones in the 1990s.

Thus, in order to respond to environmental hormonal problems and environmental regulations due to the leakage of phthalate-based plasticizers, many researchers have been conducting research in order to develop a new non-phthalate-based alternative plasticizer without phthalic anhydride used in the production of phthalate-based plasticizers, or to develop a leakage suppression technology which suppresses the leakage of phthalate-based plasticizers, thereby significantly reducing risks to human bodies and which meets environmental standards.

Meanwhile, as non-phthalate-based plasticizers, terephthalate-based plasticizers not only have an equivalent level of physical properties with phthalate-based plasticizers, but also have been spotlighted as a material free from environmental problems, so that various types of terephthalate-based plasticizers have been developed. In addition, research on developing terephthalate-based plasticizers with excellent physical properties as well as research on equipment for producing such terephthalate-based plasticizers have been actively conducted, and there has been a demand for more efficient, more economical and simpler process designs in terms of process design. Particularly, research has been actively conducted on a process in which the production of a plasticizer composition may be continuously and efficiently performed by using a continuous reactor unlike a process in which a typical batch reactor is used, and research on a method capable of more efficiently and economically operating a continuous process is also needed.

**Prior Art Document**

(Patent Document 1) Korean Patent Laid-Open Publication No. 10- 1354141
(Non-patent Document 1) Mustafizur Rahman and Christopher S. Brazel "The plasticizer market: an assessment of traditional plasticizers and research trends to meet new challenges" Progress in Polymer Science 2004, 29, 1223-1248

(Non-patent Document 2) N. R. Janjua et al. "Systemic Uptake of Diethyl Phthalate, Dibutyl Phthalate, and Butyl Paraben Following Whole-body Topical Application and Reproductive and Thyroid Hormone Levels in Humans" Environmental Science and Technology 2007, 41, 5564-5570

KR 101 663 586 B1 discloses a manufacturing method of dioctylterephthalate having low consumption of reaction energy. The manufacturing method of dioctylterephthalate comprises: a first reaction step having a plurality of reactors; and a second reaction step having a reaction temperature lower than the reaction temperature in the last reactor during the first reaction step. According to this disclsoure, a reaction conversion rate is at least 99.9%, and uniformity in the quality of products is achieved.

US 201 4/288325 A1 discloses a method for producing di(2-ethylhexyl)terephthalate (DOTP), which comprises subjecting terephthalic acid and 2-ethylhexanol to esterification in the presence of a chelate catalyst. The method of the present invention increases the reaction rate, improves the filtration efficiency of the ester product and yields DOTP with low APHA.

## DISCLOSURE OF THE INVENTION

### TECHNICAL PROBLEM

An aspect of the present invention provides a method in which the conversion rate of a reactor may be simply predicted from a flow rate of a feed introduced into the reactor and a flow rate of product water generated in the reactor in a continuous production process of a diester-based composition, and through the predicted conversion rate, a reaction in each reactor and the overall reaction in the production process may be controlled.

### TECHNICAL SOLUTION

To solve the above-mentioned object, the present invention provides a method for controlling a reaction in a continuous production process of a diester-based composition in which a dicarboxylic acid and an alcohol are reacted to produce a diester-based composition, the method including: (S1) monitoring a feed flow rate of a dicarboxylic acid and an alcohol which are introduced into a reactor and a flow rate of product water which is generated in the reactor; (S2) using the feed flow rate and the product water flow rate obtained as a result of the monitoring and pressure in the reactor to calculate a predicted conversion rate in the reactor; and (S3) controlling the temperature and the pressure of the reactor such that the calculated predicted conversion rate becomes closer to a target conversion rate; wherein the dicarboxylic acid is one or more selected from the group consisting of phthalic acid, isophthalic acid, terephthalic acid, and cyclohexane-1,4-dicarboxylic acid;

the alcohol is a C4 to C12 alcohol; and

the predicted conversion rate is calculated through Equations 1 to 4 below:

[Equation 1]

$$EC = A*FR^2 + B*FR+C$$

[Equation 2]

$$A = 7.365P + 10.415$$

[Equation 3]

$$B = -1.3163P + 10.461$$

[Equation 4]

$$C = 0.0609P + 0.0435$$

wherein, in Equation 1 to Equation 4 above,

EC is the predicted conversion rate,

FR is a ratio (product water flow rate (kg/hr)/feed feed (kg/hr)) of the flow rate of the product water to the feed flow rate of a dicarboxylic acid and the alcohol, and

P is the pressure ($kg/cm^2g$) in the reactor.

## ADVANTAGEOUS EFFECTS

When a reaction control method of the present invention is used, in a continuous production process of a diester-based composition, especially when a plurality of reactors are connected in series, and thus, it is important to control process variables in each reactor, the conversion rate of the reactor may be easily controlled from the feed flow rate at the time of initial introduction and the flow rate of product water generated in the reactor, thereby enabling the reaction control and optimization in the overall process. In addition, when a plurality of reactors are connected in series, a target conversion rate in each reactor may be appropriately set to minimize energy or raw materials unnecessarily lost, thereby enabling eco-friendly and economical process operation.

## MODE FOR CARRYING OUT THE INVENTION

Hereinafter, the present invention will be described in more detail.

It will be understood that words or terms used in the specification and claims of the present invention shall not be construed as being limited to having the meaning defined in commonly used dictionaries. It will be further understood that the words or terms should be interpreted as having meanings that are consistent with their meanings in the context of the relevant art and the technical idea of the invention, based on the principle that an inventor may properly define the meaning of the words or terms to best explain the invention.

Ester-based plasticizer compounds, which may be exemplified by a phthalate-based plasticizer, are generally produced by a typical batch reactor. Specifically, an entire amount of a carboxylic acid and an alcohol, which are reaction raw materials, is completely introduced into a batch reactor, and then a reaction is performed without the introduction of additional reaction raw materials until a desired final conversion rate is reached.

The above typical method has an advantage of stably obtaining a large amount of a plasticizer composition at a time, but has a disadvantage in that after the completion of the reaction, additional treatment such as washing the reactor with water and the like is required, so that the reactor is not actually operated for a lot of time, and thus the process equipment is not actually operated at 100%. Therefore, there has been a need to change a non-continuous production process using a typical batch reactor to a more efficient continuous production process.

However, since it is not possible to achieve a sufficient conversion rate when a continuous production process is implemented through one reactor, a plurality of reactors connected in series should be used in the continuous production process. However, when a plurality of reactors connected in series are used, appropriate reaction conditions should be created for each reactor, and also, the conversion rate in each reactor should be properly controlled to ensure excellent physical properties of a plasticizer composition to be finally produced. Therefore, there is another problem in that the difficulty of the process operation is high and many trials and errors are required to find the optimal process conditions.

As a result of the study focusing on the above problems, the inventor of the present invention has completed the present invention which is capable of predicting a conversion rate in a reactor by a small error from the flow rate of feed and product water, which can be easily checked and controlled by an operator during the operation of a continuous production process, and controlling the conversion rate of the reactor to be close to an actual target value using the predicted conversion rate.

Specifically, there is provided a reaction control method in a continuous production process of a diester-based composition, the method including S1 monitoring a feed flow rate of a dicarboxylic acid and an alcohol which are introduced into a reactor and a flow rate of product water which is generated in the reactor, S2 using the feed flow rate and the product water flow rate obtained as a result of the monitoring and pressure and temperature in the reactor to calculate a predicted conversion rate in the reactor, and S3 controlling the temperature and the pressure of the reactor such that the calculated predicted conversion rate becomes closer to a target conversion rate.

Hereinafter, the reaction control method of the present invention will be described by each step.

### Monitoring step (S1)

In order to control a conversion rate using the present invention, first, the flow rate of a feed introduced into a reactor, that is, the flow rate of a dicarboxylic acid and an alcohol, and the flow rate of product water generated in the reactor should be monitored. The flow rates of the dicarboxylic acid, the alcohol, and the product water confirmed in the present step become important factors for predicting a conversion rate of the reactor in the following step.

Specifically, the flow rate of product water may be monitored through a reflux device provided in the reactor. An esterification reaction between a dicarboxylic acid and an alcohol generates water as a by-product, and since the temperature at which the esterification reaction is performed is higher than the boiling point of water, product water in a gaseous state is continuously generated during the reaction. Although the product water does not participate in the reaction, the product water becomes a factor inhibiting heat transfer into the reactor, and thus, should be removed from the inside of the reactor, and since the product water in a gaseous state, that is, vapor, is positioned in an upper portion of the reactor, a reflux device is generally provided in the upper portion of the reactor to remove the product water.

A reflux device commonly applied in the field of process technology may be used as the reflux device. For example, the reflux device may include a water stripper column connected to the upper portion of the reactor. The product water in a gaseous state introduced into the reflux device through the upper portion of the reactor is liquefied again in the column, and some reaction raw materials vaporized together with the product water are liquefied together. A mixed solution generated during this process may be separated into reaction raw materials and product water through separate equipment, for example, a layer separator. The separated reaction raw materials may be reintroduced into the reactor to be recycled, and the product water is discharged to the outside. The flow rate of product water monitored in the present invention corresponds to the flow rate of the product water discharged to the outside during the above process, and the flow rate of the product water may be monitored with common equipment used for flow rate measurement.

Meanwhile, some of the product water may not be vaporized but remain in the reactor, or may be transferred to a next reactor together with reaction raw materials and reaction products without being discharged to the outside. However, since the temperature at which the reaction is performed is quite higher than the boiling point of water, so that the amount of the product water which is not discharged to the outside is substantially insignificant. Therefore, the flow rate of product water monitored in the present step may be substantially indicative of the entire amount of product water produced in each reactor.

The flow rate of an alcohol and a dicarboxylic acid is a value which an operator of the process may select, and the operator may select the flow rate of the dicarboxylic acid and the alcohol to be introduced into the reactor in consideration of the number and size of a reactor and the composition ratio or amount of a diester-based composition to be finally obtained.

When there is one reactor, there is no further factor to be considered regarding the flow rate of the dicarboxylic acid and the alcohol. However, when a plurality of reactors are connected in series, only a portion of a dicarboxylic acid and an alcohol initially introduced is transferred to the next reactor, so that the amount of the dicarboxylic acid and the alcohol reacted and converted to a product in the previous reactor should be further considered. Specifically, for reactors after the second reactor, the flow rate of an alcohol and a dicarboxylic acid to be introduced may be calculated and monitored through a predicted conversion rate from the previous reactor.

For example, when the flow rate of a dicarboxylic acid introduced into the first reactor is 100 kg/hr and the flow rate of an alcohol introduced thereinto is 200 kg/hr, and the flow rate of product water in the first reactor measured as a result of monitoring is 50 kg/hr and the predicted conversion rate in the first reactor calculated using Equations 1 to 4 to be described later is 50%, the flow rate of a dicarboxylic acid to be introduced into the second reactor will be 50 kg/hr, which corresponds to the remaining 50% after 50% of the flow rate of the initially introduced dicarboxylic acid of 100 kg/hr is converted, and the flow rate of an alcohol to be introduced into the second reactor will be 100 kg/hr, which corresponds to the remaining 50% after 50% of the flow rate of the initially introduced alcohol of 200 kg/hr is converted.

It is possible to obtain a predicted conversion rate of each reactor in the following step using the flow rate of each of a dicarboxylic acid and an alcohol introduced into each reactor predicted above and the flow rate of product water produced and discharged from the reactor.

Meanwhile, the molar ratio of the dicarboxylic acid and the alcohol may be 1:1.5 to 1:4, preferably 1:1.8 to 1:3.8, particularly preferably 1:1.9 to 1:3.5. The weight ratio of the dicarboxylic acid and the alcohol may vary depending on the molecular weight of the dicarboxylic acid and the alcohol. However, when the weight ratio is converted into a molar ratio, it is preferable that the weight ratio is within the above range. Since the dicarboxylic acid of one molecule reacts with the alcohol of the two molecules, the entire amount of introduced reaction raw materials is converted into a reaction product, and considering that the alcohol may be introduced excessively for the smooth progress of the reaction, it is preferable that the molar ratio of the dicarboxylic acid and the alcohol is within the above range, and when within the above range, there is an advantage in that an error between a predicted conversion rate and an actual conversion rate is particularly small.

**Predicted conversion rate calculating step (S2)**

From the flow rate of the dicarboxylic acid, the alcohol, and the product water confirmed in the above monitoring step, a predicted conversion rate (EC) of a corresponding reactor may be calculated. Specifically, the larger the ratio of the flow rate of the production water to the feed flow rate of the dicarboxylic acid and the alcohol, the more progressed the reaction is. Therefore, the flow rate ratio becomes a factor in determining a predicted conversion rate. In addition, the temperature and pressure in a reactor may shift the equilibrium of the reaction, and thus, may act as factors in determining a predicted conversion rate in each reactor.

More specifically, the predicted conversion rate may be calculated through Equations 1 to 4 below.

$$[Equation\ 1]$$

$$EC = A*FR^2 + B*FR+C$$

$$[Equation\ 2]$$

$$A = 7.365P + 10.415$$

$$[Equation\ 3]$$

$$B = -1.3163P + 10.461$$

$$[Equation\ 4]$$

$$C = 0.0609P + 0.0435$$

In Equation 1 to Equation 4 above, EC is a predicted conversion rate, FR is a ratio (product water flow rate/feed feed) of a flow rate of product water to a feed flow rate of a dicarboxylic acid and an alcohol, and P is the pressure ($kg/cm^2g$) in a reactor.

Equation 1 above is an equation for calculating a predicted conversion rate from a flow rate ratio, and Equations 2 to 4 represent that values of A, B, and C , which are coefficients or constants of Equation 1, are values varying depending on the pressure in the reactor.

In Equation 1, FR is a ratio of a flow rate of product water to a feed flow rate of a dicarboxylic acid and an alcohol, and is an indicator representing how much of the product water is produced compared to the combined flow rate of the dicarboxylic acid and the alcohol introduced into the reactor. The inventor of the present invention has found that the actual conversion rate is correlated with a secondary function result value using the FR value as a variable, and has derived Equation 1 above. In addition, Equations 2 to 4 have been derived from that the coefficients and constants in Equation 1 vary depending on the pressure in the reactor.

When a predicted conversion rate is calculated using Equations 1 to 4 above, a predicted conversion rate very close to an actual conversion rate is derived, so that the conversion rate in each reactor may be controlled thereby.

**Conversion rate controlling step (S3)**

Using a predicted conversion rate value derived from the previous step of calculating a predicted conversion rate, the following step of controlling the temperature and pressure of the reactor such that the calculated predicted conversion rate becomes closer to a target conversion rate.

A specific value of the target conversion rate may vary depending on the composition component and composition ratio in a final composition desired by a person practicing the invention, the number or size of a reactor, and specific process conditions actually applied. The person may adjust the pressure and/or temperature of each reactor such that a value of the predicted conversion rate derived in real time in the previous step becomes closer to the target conversion rate.

For example, in the case of pressure control, as confirmed from Equations 2 to 4, the coefficients and constants in Equation 1 vary depending on the pressure in the reactor, so that the pressure may be properly controlled to allow a predicted conversion rate to be higher or lower.

In the case of temperature control, the temperature control may lead to conversion rate control depending on whether the reaction is exothermic or endothermic or other reaction conditions, and by using this point, the temperature of each reactor may be adjusted such that a predicted conversion rate becomes closer to a target conversion rate.

In the case of adjusting the temperature and pressure of each reactor during the present step or setting the temperature and pressure of the reactor for the first time, the temperature in the reactor may be 180°C to 240°C, preferably 200°C to 220°C. When the temperature within the above-described range is applied, there are advantages in that it is easy to control a conversion rate and an error between a predicted conversion rate and an actual conversion rate is small.

In addition, the pressure in the reactor, that is, P in Equations 1 to 4 may be 0.1 $kg/cm^2g$ to 1.0 $kg/cm^2g$, preferably 0.2 $kg/cm^2g$ to 0.8 $kg/cm^2g$. As in the case of the temperature, when the pressure within the above-described range is applied, there are advantages in that it is easy to control a conversion rate and an error between a predicted conversion rate and an actual conversion rate is small.

### Re-monitoring step (S4)

The reaction control method of the present invention may further include Step S4 of re-monitoring a feed flow rate and a product water flow rate which are changed after the control of the temperature and the pressure of the reactor.

The reaction control method of the present invention may reduce control errors by monitoring a change in accordance with the result of control and repeating the control using the result of the monitoring as a basis for the control, rather than finishing the control once.

Specifically, even if the temperature and pressure of each reactor are controlled using a predicted conversion rate calculated through the previous step, the conversion rate for each reactor does not immediately approximate a target conversion rate. Therefore, in the reaction control method of the present invention, the result of control may be repeatedly fed back through the re-monitoring step, and accordingly, reaction control may be performed with high accuracy.

In the present re-monitoring step, the feed flow rate and the product water flow rate obtained as a result of the re-monitoring may be used as variables in Step S2, and accordingly, more accurate reaction control may be performed through the circulation of Steps S2-S3-S4-S2.

A continuous production process to which the reaction control method of the present invention may be applied may be provided with one reactor, preferably a plurality of reactors connected in series. This is because, as described above, it is difficult to achieve a sufficient conversion rate with only one reactor in a continuous process. However, it does not mean that the present invention may not be applied to a continuous production process provided with only one reactor. Equations 1 to 4 may also be effectively applied to one reactor.

The dicarboxylic acid which is applied to the reaction control method of the present invention mis one or more selected from the group consisting of phthalic acid, isophthalic acid, terephthalic acid, and cyclohexane-1,4-dicarboxylic acid, and the alcohol is a C4 to C12 alcohol. Since the dicarboxylic acid and the alcohol are of these types, there is an advantage in that an error between a predicted conversion rate and an actual conversion rate is particularly small.

Hereinafter, preferred examples are presented to aid in understanding of the present invention. However, the following examples are merely illustrative of the present invention, and are not intended to limit the scope of the present invention.

### Examples 1 to 20

Terephthalic acid and 2-ethylhexanol were selected as reaction raw materials, and the effectiveness of the reaction control method of the present invention was confirmed through ASPEN PLUS, a simulation program. Specifically, the flow rate of terephthalic acid and the flow rate of 2-ethylhexanol were respectively set to 13,000 kg/hr and 8,000 kg/hr, and when the ratio thereof was converted into a molar ratio, it was set to 1:2. The flow rate of product water was calculated from a conversion rate. One CSTR reactor was used as the reactor, and how an actual conversion rate varies for a total of 20 examples having different pressures and temperatures, and a predicted conversion rate calculated and obtained from Equations 1 to 4 for each case are summarized in Table 1 below.

[Table 1]

| Reaction pressure ($kg/cm^2g$) | Flow rate ratio and conversion rate | Reaction temperature (°C) | | | | |
|---|---|---|---|---|---|---|
| | | 200 | 205 | 210 | 215 | 220 |
| 0.8 | Example number | 1 | 2 | 3 | 4 | 5 |
| | Flow rate ratio (product water/ feed) | 0.0427 4 | 0.0470 4 | 0.0510 3 | 0.0547 0 | 0.0580 4 |
| | Actual conversion rate (%) | 52.401 | 57.072 | 61.465 | 65.553 | 69.309 |
| | Predicted conversion rate (%) | 52.415 | 57.088 | 61.479 | 65.565 | 69.324 |
| | Conversion rate difference | 0.014 | 0.016 | 0.014 | 0.012 | 0.015 |

(continued)

| Reaction pressure (kg/cm²g ) | Flow rate ratio and conversion rate | Reaction temperature (°C) | | | | |
|---|---|---|---|---|---|---|
| | | 200 | 205 | 210 | 215 | 220 |
| 0.6 | Example number | 6 | 7 | 8 | 9 | 10 |
| | Flow rate ratio (product water/feed) | 0.0430 9 | 0.0473 3 | 0.0512 7 | 0.0549 1 | 0.0582 1 |
| | Actual conversion rate (%) | 52.426 | 57.089 | 61.478 | 65.566 | 69.318 |
| | Predicted conversion rate (%) | 52.435 | 57.102 | 61.489 | 65.576 | 69.331 |
| | Conversion rate difference | 0.009 | 0.013 | 0.011 | 0.010 | 0.013 |
| 0.4 | Example number | 11 | 12 | 13 | 14 | 15 |
| | Flow rate ratio (product water/feed) | 0.0434 3 | 0.0476 1 | 0.0515 1 | 0.0551 1 | 0.0583 8 |
| | Actual conversion rate (%) | 52.446 | 57.104 | 61.497 | 65.578 | 69.326 |
| | Predicted conversion rate (%) | 52.455 | 57.115 | 61.507 | 65.587 | 69.337 |
| | Conversion rate difference | 0.009 | 0.011 | 0.010 | 0.009 | 0.011 |
| 0.2 | Example number | 16 | 17 | 18 | 19 | 20 |
| | Flow rate ratio (product water/feed) | 0.0437 7 | 0.0478 9 | 0.0517 5 | 0.0553 0 | 0.0585 5 |
| | Actual conversion rate (%) | 52.468 | 57.122 | 61.511 | 65.586 | 69.335 |
| | Predicted conversion rate (%) | 52.479 | 57.135 | 61.521 | 65.596 | 69.346 |
| | Conversion rate difference | 0.011 | 0.013 | 0.010 | 0.010 | 0.011 |

As can be seen from Table 1, the predicted conversion rates calculated through Equations 1 to 4 of the present invention showed values almost similar to the actual conversion rates, and the error range was only 0.01%. Therefore, it was confirmed that the conversion rate of the reactor may be predicted with high accuracy.

Therefore, a conversion rate is predicted through the present invention, and then, using the predicted conversion rate, the conversion rate of a reactor may be changed to be close to a target conversion rate through the control of temperature and pressure. In addition, whether an actual conversion rate has become close to the target conversion rate as a result of the control of temperature and pressure may also be confirmed through monitoring after the control and the calculation of a predicted conversion rate, so that when the present invention is used, it is possible to simply provide continuous feedback on the process.

**Claims**

1.  A method for controlling a reaction in a continuous production process of a diester-based composition in which a dicarboxylic acid and an alcohol are reacted to produce a diester-based composition, the method comprising:

    (S1) monitoring a feed flow rate of the dicarboxylic acid and the alcohol which are introduced into a reactor and a flow rate of product water which is generated in the reactor;
    (S2) using the feed flow rate and the product water flow rate obtained as a result of the monitoring and pressure in the reactor to calculate a predicted conversion rate in the reactor; and
    (S3) controlling the temperature and the pressure of the reactor such that the calculated predicted conversion rate becomes closer to a target conversion rate;
    wherein the dicarboxylic acid is one or more selected from the group consisting of phthalic acid, isophthalic acid, terephthalic acid, and cyclohexane-1,4-dicarboxylic acid;
    the alcohol is a C4 to C12 alcohol; and
    the predicted conversion rate is calculated through Equations 1 to 4 below:

[Equation 1]

$$EC = A*FR^2 + B*FR+C$$

[Equation 2]

$$A = 7.365P + 10.415$$

[Equation 3]

$$B = -1.3163P + 10.461$$

[Equation 4]

$$C = 0.0609P + 0.0435$$

wherein, in Equation 1 to Equation 4 above,
EC is the predicted conversion rate,
FR is a ratio (product water flow rate (kg/hr)/feed feed (kg/hr)) of the flow rate of the product water to the feed flow rate of a dicarboxylic acid and the alcohol, and
P is the pressure ($kg/cm^2g$) in the reactor.

2. The method of claim 1, further comprising (S4) re-monitoring the feed flow rate and the product water flow rate which are changed after the control of the temperature and the pressure of the reactor.

3. The method of claim 2, wherein the feed flow rate and the product water flow rate obtained as a result of the re-monitoring are used as variables in Step S2.

4. The method of claim 1, wherein the molar ratio of the dicarboxylic acid and the alcohol is 1:1.5 to 1:4.

5. The method of claim 1, wherein the continuous production process is provided with a plurality of reactors connected in series.

6. The method of claim 1, wherein the temperature in the reactor is 180°C to 240°C.

7. The method of claim 6, wherein the temperature in the reactor is 200°C to 220°C.

8. The method of claim 1, wherein P is 0.1 $kg/cm^2g$ to 1.0 $kg/cm^2g$.

9. The method of claim 8, wherein P is 0.2 $kg/cm^2g$ to 0.8 $kg/cm^2g$.


**Patentansprüche**

1. Verfahren zum Steuern einer Reaktion in einem kontinuierlichen Herstellungsverfahren einer Zusammensetzung auf Diesterbasis, in der eine Dicarbonsäure und ein Alkohol umgesetzt werden, um eine Zusammensetzung auf Diesterbasis herzustellen, wobei das Verfahren umfasst:

   (S1) Überwachen einer Zufuhrströmungsrate der Dicarbonsäure und des Alkohols, die in einen Reaktor eingeführt werden, und einer Strömungsrate von Produktwasser, das in dem Reaktor erzeugt wird;
   (S2) Verwenden der Zufuhrströmungsrate und der Produktwasserströmungsrate, die als ein Ergebnis der Überwachung erhalten werden, und des Drucks in dem Reaktor, um eine vorhergesagte Umwandlungsrate in dem Reaktor zu berechnen; und
   (S3) Steuern der Temperatur und des Drucks des Reaktors, so dass die berechnete vorhergesagte Umwandlungsrate näher an eine Zielumwandlungsrate herankommt;
   wobei die Dicarbonsäure eine oder mehrere ist, die aus der Gruppe ausgewählt sind, die aus Phthalsäure, Isophthalsäure, Terephthalsäure und Cyclohexan-1,4-dicarbonsäure besteht;
   der Alkohol ein C4- bis C12-Alkohol ist; und

die vorhergesagte Umwandlungsrate durch die folgenden Gleichungen 1 bis 4 berechnet wird:

[Gleichung 1]

$$EC = A*FR^2 + B*FR+C$$

[Gleichung 2]

$$A = 7,365P + 10,415$$

[Gleichung 3]

$$B = -1,3163P + 10,461$$

[Gleichung 4]

$$C = 0,0609P + 0,0435$$

wobei in Gleichung 1 bis Gleichung 4 oben
EC die vorhergesagte Umwandlungsrate ist,
FR ein Verhältnis (Produktwasserströmungsrate (kg/h)/Zufuhrzufuhr (kg/h)) der Strömungsrate des Produktwassers zu der Zufuhrströmungsrate einer Dicarbonsäure und des Alkohols ist, und
P der Druck (kg/cm$^2$g) in dem Reaktor ist.

2. Verfahren nach Anspruch 1, ferner umfassend (S4) erneutes Überwachen der Zufuhrströmungsrate und der Produktwasserströmungsrate, die nach der Steuerung der Temperatur und des Drucks des Reaktors geändert werden.

3. Verfahren nach Anspruch 2, wobei die Zufuhrströmungsrate und die Produktwasserströmungsrate, die als ein Ergebnis der erneuten Überwachung erhalten werden, als Variablen in Schritt S2 verwendet werden.

4. Verfahren nach Anspruch 1, wobei das Molverhältnis der Dicarbonsäure und des Alkohols 1:1,5 bis 1:4 beträgt.

5. Verfahren nach Anspruch 1, wobei das kontinuierliche Herstellungsverfahren mit einer Vielzahl von Reaktoren versehen ist, die in Reihe geschaltet sind.

6. Verfahren nach Anspruch 1, wobei die Temperatur in dem Reaktor 180 °C bis 240 °C beträgt.

7. Verfahren nach Anspruch 6, wobei die Temperatur in dem Reaktor 200 °C bis 220 °C beträgt.

8. Verfahren nach Anspruch 1, wobei P 0,1 kg/cm$^2$ g bis 1,0 kg/cm$^2$ g beträgt.

9. Verfahren nach Anspruch 8, wobei P 0,2 kg/cm$^2$ g bis 0,8 kg/cm$^2$ g beträgt.

**Revendications**

1. Procédé de régulation d'une réaction dans un procédé de production en continu d'une composition à base de diester dans lequel un acide dicarboxylique et un alcool sont mis à réagir pour produire une composition à base de diester, le procédé consistant à :

(S1) surveiller un débit d'alimentation de l'acide dicarboxylique et de l'alcool qui sont introduits dans un réacteur et un débit de l'eau produite qui est générée dans le réacteur ;
(S2) utiliser le débit d'alimentation et le débit d'eau produite obtenus à la suite de la surveillance et la pression dans le réacteur pour calculer un taux de conversion prévu dans le réacteur ; et
(S3) réguler la température et la pression du réacteur de telle sorte que le taux de conversion prévu calculé se rapproche d'un taux de conversion cible ;
dans lequel l'acide dicarboxylique est un ou plusieurs acides sélectionnés dans le groupe constitué de l'acide phtalique, l'acide isophtalique, l'acide téréphtalique et l'acide cyclohexane-1,4-dicarboxylique ;
l'alcool est un alcool en C4 à C12 ; et
le taux de conversion prévu est calculé par les Équations 1 à 4 ci-dessous :

[Équation 1]

$$EC = A*FR^2 + B*FR+C$$

[Équation 2]

$$A = 7,365P + 10,415$$

[Équation 3]

$$B = -1,3163P + 10,461$$

[Équation 4]

$$C = 0,0609P + 0,0435$$

dans lequel, dans l'Équation 1 à l'Équation 4 ci-dessus,
EC est le taux de conversion prévu,
FR est un rapport (débit d'eau produite (kg/h)/vitesse d'alimentation (kg/h)) du débit de l'eau produite au débit d'alimentation d'un acide dicarboxylique et de l'alcool, et
P est la pression ($kg/cm^2g$) dans le réacteur.

2. Procédé selon la revendication 1, consistant en outre à (S4) resurveiller le débit d'alimentation et le débit d'eau produite qui ont changé après la régulation de la température et de la pression du réacteur.

3. Procédé selon la revendication 2, dans lequel le débit d'alimentation et le débit d'eau produite obtenus à la suite de la re-surveillance sont utilisés comme variables à l'étape S2.

4. Procédé selon la revendication 1, dans lequel le rapport molaire de l'acide dicarboxylique et de l'alcool est 1:1,5 à 1:4.

5. Procédé selon la revendication 1, dans lequel le processus de fabrication en continu est fourni avec une pluralité de réacteurs connectés en série.

6. Procédé selon la revendication 1, dans lequel la température dans le réacteur est 180°C à 240°C.

7. Procédé selon la revendication 6, dans lequel la température dans le réacteur est 200°C à 220°C.

8. Procédé selon la revendication 1, dans lequel P est 0,1 $kg/cm^2g$ à 1,0 $kg/cm^2g$.

9. Procédé selon la revendication 8, dans lequel P est 0,2 $kg/cm^2g$ à 0,8 $kg/cm^2g$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200068019
- KR 101354141
- KR 101663586 B1
- US 2014288325 A1

**Non-patent literature cited in the description**

- **MUSTAFIZUR RAHMAN ; CHRISTOPHER S.BRA-ZEL.** The plasticizer market: an assessment of traditional plasticizers and research trends to meet new challenges. *Progress in Polymer Science,* 2004, vol. 29, 1223-1248
- **NR JANJUA et al.** Systemic Uptake of Diethyl Phthalate, Dibutyl Phthalate, and Butyl Paraben Following Whole-body Topical Application and Reproductive and Thyroid Hormone Levels in Humans. *Environmental Science and Technology,* 2007, vol. 41, 5564-5570
- **MUSTAFIZUR RAHMAN ; CHRISTOPHER S. BRA-ZEL.** The plasticizer market: an assessment of traditional plasticizers and research trends to meet new challenges. *Progress in Polymer Science,* 2004, vol. 29, 1223-1248
- **N. R. JANJUA et al.** Systemic Uptake of Diethyl Phthalate, Dibutyl Phthalate, and Butyl Paraben Following Whole-body Topical Application and Reproductive and Thyroid Hormone Levels in Humans. *Environmental Science and Technology,* 2007, vol. 41, 5564-5570